# EUROPEAN PATENT APPLICATION

(11) **EP 4 134 970 A1**
(43) Date of publication of application: **15.02.2023**
(21) Application number: 21190411.5
(22) Date of filing: 09.08.2021
(51) Int. Cl.: G16H 30/20, G16H 40/63

(54) **METHOD FOR SELECTING AND DISPLAYING VIRTUAL PRESENTATIONS OF A BODY PORTION GENERATED IN MEDICAL STUDIES**

(71) Applicant: Ai Medical AG, 8702 Zollikon (CH)
(72) Inventor: Federau, Christian, 8702 Zollikon (CH)
(74) Representative: Frei Patent Attorneys

(57) **Abstract**

The invention relates to the field of medical imaging technology and concerns a method and a system for selecting, ordering, displaying and/or navigating virtual presentations, in particular images, of a body portion that are generated during medical examinations (studies). The method comprises a step S5 of providing in a user interface 1 a list 5 of studies, a list 8 of presentations and a presentation 3 of the body portion. The list 5 of studies comprises items that are characteristic for a kind of study. The list 8 of presentations comprises items that are characteristic for a kind of presentation. The presentation 3 displayed in the user interface 1 depends on the item selected in the list 5 of studies and the item selected in the list 8 of presentations.

## Description

### FIELD OF THE INVENTION

The invention relates to the field of medical imaging technology and concerns methods and devices for selecting, ordering, displaying and/or navigating virtual presentations of a body portion. The presentations are generated during a medical examination, also called study, for example by acquiring the presentations directly or by generating them from raw data or "raw images" acquired during a study, wherein presentations of different kinds, in particular presentations optimized for visualizing different tissue or tissues and/or pathology or pathologies, are generated typically in a study. Examples of medical examinations (studies) to which the present invention relates are computer tomography (CT) examination and magnetic resonance (MR) examination. 2D images, 3D images, arrangements of 2D images or 3D images, scrollable images and movies of the body portion are examples virtual presentations of the body portion, wherein the virtual presentations are generated for and of kind suitable for a medical purpose, in particular for assessing a medical condition.

### BACKGROUND OF THE INVENTION

A medical examination (study) to which the present invention relates typically provides of a plurality of virtual presentations of a body portion, wherein the presentations are suitable to be provided to a user, for example a practitioner, for assessing a medical condition of the body portion shown in the presentations.

A virtual presentation of the body portion may comprise or be a 2D image of the body portion, a 3D image of the body portion, a volume rendered image of the body portion, a spatial arrangement of such images, or a movie of the body portion, for example. A 2D image generated with a top view imaging technique and a slice or projection of a 3D representation of the body portion are examples of a 2D image of the body portion. A 3D visualization of the body portion or a scrollable image of the body portion are examples of a 3D image of the body portion. A movie that shows different planes of the body portion in a consecutive manner is an example of a movie presentation of the body portion.

Amongst other things, the presentations provided in a study may differ in the clinical purpose they are optimized for. In simple terms, they may differ in the tissue or tissues and/or pathology or pathologies that may be examined best when studying a presentation.

The manner this optimization is done depends on the examination method.

For example, in the case of a CT examination, the examination comprises, in many cases consists of, a scan in which raw data, usually a volume of voxels, is acquired. Different presentations of the scanned body portion are generated then by reconstructing presentations from the raw data with different reconstruction kernels or different images filters followed by windowing. During reconstruction and in particular during windowing, presentations are generated that are optimized for specific clinical purposes.

For example, in the case of a magnetic resonance examination an examination (study) typically comprises, usually consists of, a plurality of acquisitions that are carried out with different settings. A setting used during an MRI acquisition is also called an MRI sequence or contrast. The acquisitions carried out using different settings result in presentations of the examined body portion that are optimized for a specific clinical purpose. In other words, the presentations of an MRI examination base on acquirements with different settings, in particular different pulse sequences and pulsed field gradients, for addressing a specific clinical purpose. Fluid Attenuated Inversion Recovery (FLAIR), T1-weighted pulse sequence, T2-weighted pulse sequence, or sequences of the diffusion-weighted imaging group are examples of different MRI sequences.

Independent of the examination method, a presentation of a study has finally a contrast that is optimized for a specific clinical purpose and that is different from the contrast of another, potential or generated, presentation of the study. Thereby, the contrast is related to the settings used during acquisition of the presentation or of data on which the presentation bases and/or to the manner the acquired data is processed.

For CT examination for example, contrasts can be defined according to the contrast-window, for example the center and width of the region of the Hounsfield scale, such as soft-tissue contrast and bone contrast.

For MR examination for example, contrasts can be defined according to the MRI sequence used during acquisition, such that FLAIR, T1-weighted, T2-weighted, proton-density and diffusion-weighted are examples of different contrasts.

In more general terms, each presentation of a study can be related to at least one characteristic used during generation of the presentation, in particular during acquisition of raw data or the presentation and/or during processing the raw data on which the presentation bases, said characteristic being representative for a specific kind of presentation, in particular for the clinical purpose the presentation is optimized for.

It is an insight of the invention, that said characteristic can be used in an efficient and resource-friendly manner for selecting, ordering, displaying and navigating the presentations of a plurality of studies.

Medical images software according to the state-of-the-art typically present the studies and presentations in chronological order as a list, with a name (for example the sequence name for MR, the reconstruction kernel name for CT) and/or a small representative image. This manner of presenting the studies and presentations is highly inefficient because the practitioner must first open the study she/he is interested in, then find the presentation she/he is interested in, and then use some kind of display tool, for example by selecting the presentation or by using a drag and drop function. It is evident that the practitioner, by this, spends a significant amount of time searching for the presentation she/he wants to look at, and that the practitioner has to repeat these working steps every time she/he wants to look at a new presentation or a new study.

### SUMMARY OF THE INVENTION

It is an object of the invention to provide methods and devices that overcome at least some of the drawback of methods and devices according to the state-of-the-art.

In particular, it is an object of the invention to provide a method and a system that supports a practitioner in analyzing the presentations of a body portion generated during a medical examination (study). In particular, it is an object of the invention to provide a method and a system for selecting and displaying in an efficient manner the presentations of a study or studies of a selected patient.

Further, it is an object of the invention to provide a method and a system for navigating, this means switching a displayed presentation to another presentation corresponding to another study and/or another kind of presentation, in an efficient manner.

Further, it is an object of the invention to provide a method for ordering studies and their presentations in a way to further improve the efficiency of the selection, display, and navigation of the presentations by the practitioner.

The method is a computer-implemented method or a computer supported method at least. Therefore, it is a further object of the invention to provide a related computer program, a related computer readable medium, a related computer-readable signal, and a related data carrier signal.

At least one of the objects is achieved by the invention described in the following and/or the invention claimed in the claims.

A method according to the invention is a method for at least one of selecting, ordering, displaying and navigating virtual presentations of a body portion, wherein the virtual presentations are generated during medical studies, for example during a radiologic, magnetic resonance imaging (MRI), computer tomographic (CT), sonographic, or photographic study.

The method for at least one of selecting, ordering, displaying and navigating virtual presentations of body portions generated in medical studies, in particular for selecting a displaying, comprises the following steps:
- A step of providing a list of studies comprising at least one item that is characteristic for a kind of study.

For example, a list of studies may be generated, wherein the list of studies comprises items comprising a description defining a corresponding study.

The size of the list of studies can be different than the number of studies available for one patient.

Usually, the items of the list of studies are unique items, this means that the list of studies comprises unique items, only. In other words, each item of the list of studies appears only once in the list of studies.

Further, the list of studies is provided in a manner that each item may correspond maximally to one study related to the medical case to be examined.

In an embodiment, the items of the list of studies are suitable for ordering the studies of a patient, or a selection thereof, in chronological order.

A first kind of study may differ from a second kind of study in the date the study was generated, for example.

In an embodiment, each item of the list of studies is or comprises the date of a corresponding study, or the items represent an increasing or decreasing numbering.

The list of studies can be provided in an automated manner or it can be defined by a user, for example a practitioner.

In an embodiment, the list of studies is provided in an automated manner. For example, upon selecting a patient by the user, the list of studies is generated by reading the study dates of the studies available for the user and optionally for the body portion to be examined.
- A step of providing a list of presentations comprising at least one item that is characteristic for a kind of presentation.

For example, a list of presentations may be generated, wherein the list of presentations comprises items comprising a description defining a corresponding presentation.

A first kind of presentation may differ from a second kind of presentation in the settings used during acquisition of the presentations or raw data and/or features used during processing of the raw data, for example as disclosed above with respect to CT and MR examination.

The size of the list of presentations can be different than the number of different kinds of presentations available for the patient.

Usually, the items of the list of presentations are unique items. In other words, each item of the list of presentations appears only once in the list of presentations.

However, there may be embodiments of the method in which more than one list of presentations is provided, for example one list of presentations for each study assigned to an item of the list of studies. In this case, an item in a first list of presentation is usually identical to an item in a second list of presentations, at least if the kind of presentation to which the item refers has been generated in both studies. In other words, the list(s) of presentations provided during the method may comprise items that are unique within a specific list of presentations, but the items may not be unique when considering another list of presentations that is provided during the method.

In an embodiment, the items of the list of presentations refer to at least one of the settings available or used during acquisition of raw data or presentations and/or features available or used during processing the acquired raw data or the acquired presentations. Then, a presentation that is generated by using a specific setting or specific settings and/or a specific feature or specific features can be identified and accessed if the list of presentations comprises an item related to said specific setting or specific settings and/or to said specific feature or specific features and the presentation was assigned to said item. In other words, the items of the list of presentations are suitable for identifying different presentations, this means different kinds of presentations.

The step of assigning presentations to an item of the list of presentations is described below in detail.

In an embodiment, the items are or comprise hints towards the settings and/or features used for optimizing a presentation for a specific clinical purpose.

In particular, the items, more precisely their labeling, may name or indicate the available or used contrasts in the sense as disclosed above. In the case of CT and MR examination, the label of the item may comprise terms such as FLAIR or T1-weighted for MRI, or soft-tissue or bone window for CT.

In an embodiment, the list of presentations is a list of contrasts.

The list of presentations, in particular if it is a list of presentations that is appropriate for a specific type of study and/or a specific medical case, can be provided in an automated manner or it can be defined by the user.

In an embodiment, the list of presentations is provided in an automated manner, for example by reading out meta data.
- A step of assigning a study to an item of the at least one item of the list of studies.

Usually, more than one study is assigned to the list of studies if a plurality of studies for the patient and the body portion to be examined is available. In particular, all studies of the patient and the body portion to be examined that are available may be assigned to an item of the list of studies or a selection of said studies.

The assignment of studies to the list of studies is such that maximal one study is assigned to each item of the list of studies. Typically, one study is assigned to every item of the list of studies.
- A step of assigning a presentation, for example each presentation or a selection of the presentations, provided in the study or, as the case may be, studies assigned to an item of the at least one item of the list of studies to an item of the at least one item of the list of presentations.

The assignment of presentations to the lists of presentations (to the lists of presentations if more than one list of presentations is provided, for example one for each study assigned to an item in the list of studies) is such that maximal one presentation is assigned to each item of the (each) list of presentations. However, there may be one or more items to which no presentation is assigned in cases in which no assignment is possible. In the example that the items of the lists of presentations concern contrasts, one of the contrasts might for example not have been used for generating a presentation.

In summary, the presentations available for a patient can be assigned to one of the items of the list of studies or to none by assigning or not assigning the study to which a presentation belongs to an item of the list of studies, and to one of the items of a list of presentations or to none by assigning or not assigning the presentation to an item of the list of presentations.

In an embodiment, a mechanism is provided to ensure that maximal one study of the patient is assigned to an item of the list of studies and that maximal one presentation is assigned to an item of the list of presentations. An example of such a mechanism is if there is more than one presentation that could be assigned to one item of a list of presentations, then the presentation with the later date is assigned.

In an embodiment, a set of rules is used in the step of assigning a study to an item of the list of studies and/or in the step of assigning a presentation to an item of the list of presentations.

For example, a list of characteristics, this means of kinds of studies and/or of kinds of presentations, that are to be assigned (list of including characteristics in the following) can be provided, for example by the user.

The method according to this embodiment can comprise a step of scanning the name of a study and/or of a presentation or scanning other metadata related to the study and/or presentation for a characteristic, in particular for a characteristic comprised in the list of including characteristics.

The assignment of the study or presentation can then depend on whether the scanned name or metadata comprises at least one characteristic that corresponds to at least one characteristic of the list of including characteristics.

Alternatively or in addition, a list of characteristics, this means of kinds of studies and/or of kinds of presentations, that are not to be assigned (list of excluding characteristics in the following) can be provided and used in the step of assigning a study and/or in the step of assigning a presentation.

In embodiments, the characteristics of the list of including characteristics and/or of the list of excluding characteristics as well as the characteristic or characteristics for which the name or metadata is scanned are given as terms, this means as a sequence of letters and/or numbers. In other words, the list of including characteristics may be a list of including terms and the list of excluding characteristics may be a list of excluding term.

In an embodiment, the scanned name or metadata must comprise any term comprised in the list of including terms or a subset thereof for the study or presentation related to said name or metadata being assigned.

In an embodiment, a plurality of lists of including terms is provided and the scanned name or metadata must comprise, for each of the provided lists of including terms, at least one term that is comprised in the list of including terms for the study or presentation related to said name or metadata being assigned.

For example, in case the items of the list of presentations relate to contrasts, the assignment can be determined by scanning the name, for example the sequence name in the case of MR examination, of the presentation, said name is normally saved in the header of the presentation, and by assigning the presentation if a term found during scanning corresponds to an item of a predefined list of including terms (contrasts), while at the same time the term found corresponds to none of the items of a list of excluding terms (contrast).

Alternatively or in addition, a model of an artificial intelligence (AI), such as a neural network, may be used in the step of assigning a study to an item of the list of studies and/or in the step of assigning a presentation to an item of the list of presentations.

In particular, the model of the AI may be trained to classify a presentation according to the items of the list of presentations. For example, the model may be trained to recognize the contrast of the presentation.

In an embodiment, the method comprises a step of correcting the assignments done according to any embodiment disclosed by the user. Therefore, a user interface, in particular the user interface according to any embodiment disclosed below, may comprise tools configured for correcting assignments by the user, for example a pop-up view, such as a preference panel.
- A step of providing to a user via a user interface, the list of studies or an adapted list of studies and the lists of presentations or an adapted list of presentations.

The adapted list of studies may comprise only the items of the list of studies to which a study was assigned and/or it may comprise only items related to studies selected by the user. In addition or alternatively, the adapted list of studies may be organized (ordered) diffrently than the list of studies.

The adapted list of presentations may comprises only the items of the list of presentations to which a presentation was assigned and/or it may comprise only items related to kind of presentations selected by the user. In addition or alternatively, the adapted list of presentations may be organized (ordered) diffrently than the list of presentations.

In embodiments in which more than one list of presentations is provided during the method, the lists of presentations may comprise the same set of items, independent whether a presentation was assigned to an item or not. This makes navigating through different studies and identification of the presentation displayed in the image viewer more intuitive.

In embodiments in which more than one list of presentations is provided, for example one for each study assigned to an item of the list of studies, one, in particular only one, of the provided lists of presentations may be provided to the user via the user interface.

For example, the list of presentations that is provided for the most recent study assigned to an item of the list of studies is provided to the user in an automated manner. The item of the list of study corresponding to the most recent study may be highlighted for indicating that the list of presentations corresponding to the most recent study is provided to the user, when the list of studies is provided to the user.

The user interface comprises an image viewer, a first view for the list of studies or the adapted list of studies, and a second view for the list of presentations or the adapted list of presentations.

The first view and the second view are configured to allow the selection of an item of the list shown in the first view and the list shown in the second view, respectively.

The first view, the second view and the image viewer are configured for the presentation to be displayed in the image viewer that belongs to the study and kind of presentation indicated by the item selected in the list displayed in the first view and the item selected in the list displayed in the second view.
- A step of selecting a presentation to be displayed in the image viewer by selecting an item of the list shown in the first view and by selecting an item of the list shown in the second view.

The selected presentation to be displayed is displayed immediately after selection and in an automated manner, usually.

Usually, the items of the list shown in the second view are linked to the assigned presentations of the study that is assigned to the selected item of the list shown in the first view.

This also means that the presentation to which an item of the list shown in the second view refers depends on the item selected in the list shown in the first view. This is in particular the case if the item of the list shown in the second view refers to a kind of presentation that is present in a first study assigned to a first item of the list shown in the first view and in a second study assigned to a second item of the list shown in the in the first view.

Further, this means that the presentation to which an item of the list shown in the second view refers changes if the item selected in the list shown in the first view changes.

In embodiments in which a list of presentations is provided for each study assigned to an item of the at least one item of the list of studies and in which the presentations of an assigned study or a selection of the presentations of the assigned study are assigned to the list of presentations provided for said study, the (adapted, as the case may be) list of presentations that is provided to the user is the (adapted) list of presentations provided for the study selected in the (adapted, as the case may be) list of studies shown in the first view.

In other words, the list shown in the second view is the list of presentations that corresponds to the study assigned to the selected item of the list shown in the first view.

Due to the fact that the presentation displayed in the image viewer belongs to the study and kind of presentation indicated by the item selected in the list displayed in the first view and the item selected in the list displayed in the second view, a change in the item selected in the list displayed in the first view, this means in the (adapted, as the case may be) list of studies, causes a change of the presentation displayed in the image viewer to a presentation belonging to the newly selected study and being of the same kind, this means having the same characteristic with respect to the characteristics indicated by the items of the list shown in the second view, this means the (adapted, as the case may be) list of presentations, (no change of the item selected in the list shown in the second view) as the presentation displayed before changing the item selected in the list shown in the first view.

Due to the fact that the presentation displayed in the image viewer belongs to the study and kind of presentation indicated by the item selected in the list displayed in the first view and the item selected in the list displayed in the second view, a change in the item selected in the list displayed in the second view, this means the (adapted, as the case may be) list of presentations, causes a change of the presentation displayed in the image viewer to a presentation of the newly selected kind of presentation and belonging to the same study (no change of the item selected in the list shown in the first view) as the presentation displayed before changing the item selected in the list shown in the second view.

In an embodiment, the selection of another item of the list shown in the first view than the one currently selected occurs at a fixed item in the list shown in the second view, and the selection of another item of the list shown in the second view than the one currently selected occurs at a fixed item in the list shown in the first view.

For example, in the case that the items of the list of presentations correspond to contrasts, the navigation in the list of studies results in displaying presentations having the same contrast but belonging to different studies. This results in an efficient navigation for the user, permitting the comparison of presentations with the same contrast of different studies with a one-click operation, for example using a keyboard shortcut, pressing on a toolbar item, or selecting an item in the first view.

For example, all the available items of the list of studies might be displayed as a selectable list in the first view. For example, the selectable list may display all available exam dates of a currently selected patient. The available exam dates may be displayed in chronological order. The item currently selected in the list shown in the first viewmay be highlighted. Similarly, all the available items of the list of presentations might be displayed as a selectable list in the second view. For example, the selectable list may display all available contrasts of the currently selected study. The item currently selected in the list displayed in the second view may be highlighted. The user may then change the currently selected item in both the list provided in the first view and the list provided in the second view by clicking on another item of the lists. The presentation corresponding to the selected item of the list shown in the first view and to the selected item of the list shown in the second view is displayed in the image viewer, and the user might display another presentation corresponding to another item of the list shown in the first view and/or of the list shown in the second view by clicking on that specific item in the corresponding list.

Alternatively, or in addition, to selecting an item of the list shown in the first view and/or of the list shown in the second view by clicking on the item, the user interface may comprise a toolbar item configured to navigate in the list shown in the first view and to change the currently displayed study. In particular, the toolbar item may be configured to move up and/or down in the list shown in the first view.

Similarly, the user interface may comprise a toolbar item configured to navigate in the list shown in the second view and to change the currently displayed kind of presentation. In particular, the toolbar item may be configured to move up and/or down in the list shown in the second view.

Alternatively, or in addition, a system on which the method is carried out may be configured to accept one or more keyboard shortcuts that permit to navigate in the list shown in the first view and/or the list shown in the second view and to change the currently selected item of the list shown in the first view and/or of the list shown in the second view. In particular, the system may be configured to accept one or more keyboard shortcuts for moving up and/or down in the list shown in the first view and/or the list shown in the second view.

In an embodiment, the step of assigning studies to items of the list of studies, the step of assignging presentations to items of the lists of presentations, and the step of providing to a user the (adapted, as the case may be) list of study and the (adapted, as the case may be) list of presentations are automated steps. This means these steps are carried out in an automated manner, except an optional initialization by the user.

Optionally, the step of providing a list of studies and/or the step of providing a list of presentations may be an automated step.

Optionally, at least one of the step of providing a list of studies and the step of providing a list of presentations may be a semi-automated step. For example, at least one of a proposal for a list of studies and a proposal for a list of presentations may be provided to the user in an automated manner and the user may adapt or approve the proposed list of studies and/or the proposed list of presentations.

In an embodiment, the list shown in the first view, this means the (adapted, as the case may be) list of studies provided to the user, is an ordered (organized) list, and the list shown in the second view, this means the (adapted, as the case may be) list of presentations provided to the user, is an ordered (organized) list.

A list is ordered (organized) when it is given an order, for example through an automatic ordering method or through user interactions.

For example, the list of studies may be ordered in chronological ascending or descending study date, this means the date the study was generated.

For example, the list of presentations may be assigned a fixed order. In the case that the items of the list of presentations correspond to contrasts, the fixed order may be given by placing the FLAIR first, then the T1-weighted second, etc., for example.

In an embodiment, the user interface may provide a tool for amending the order of the list of studies and/or the order of the list of presentations.

Usually, at least the presentations assigned to an item of the list of presentations are coregistered to one another or to a reference presentation. This means that the presentations have been or are transformed in a manner that the similarity of the presentations is maximized with respect to one another or with respect to a reference presentation.

For example, the presentations are coregistered to a reference presentation, such as a template presentation, wherein the template presentation can be one of the presentations assigned (or any other presentation generated for the body portion), and hence to one another or the presentations are coregistered directly to one another. Coregistration might improve the ease to compare presentations.

Co-registration may be carried out with respect to presentation ("image") orientation, presentation position (image position, slice number), position and orientation of the body portion shown, zoom, presentation ("image") contrast etc.

For example, coregistration may comprise a step of transforming a presentation to the template presentation, and a step of resampling the transformed presentation to the template presentation, for example using interpolation, so that the voxel positions of the transformed presentation match the voxel positions of the template presentation.

Thanks to co-registration, a replacement presentation that replaces a presentation that is displayed actually in the image viewer may be nearly identical in terms of presentation orientation, presentation position (slice number), position and orientation of the body portion shown, zoom, presentation ("image") contrast etc. In other words, the replacement presentation is nearly identical to the actually displayed presentation, exept for differences that originate from the fact that the replacement presentation belongs to a different study and/or a different kind of presentation than the presentation that is displayed actually.

In an embodiment at least the presentations assigned to an item of the list of presentations are coregistered to a reference presentation, wherein the reference presentation is determined by an algorithm from a set of presentations that comprises all presentations assigned to an item of the list (or lists, as the case may be) of presentations. For example the reference presentation is determined by the algorithm from all presentations of all studies concerning a specific body portion of a specific patient.

For example, the reference presentation may be determined in an automated manner by using a set of rules or by a machine learning algorithm.

Alternatively, the reference presentation may be determined by the user or a reference presentation determined in an automated manner may be corrected by the user. In this case, the user may be different from the user that examines the presentations. For example, it may be the person that is responsible for operating the medical imaging system used for generating the studies.

The reference presentation can be modified by the user, for example through a selection in a preference panel.

This reference presentation might for example be defined as a specific contrast, such as the FLAIR, of the current study.

In an embodiment, the method comprises at least one of the following steps carried out by the user, in particular by the user interface comprising tools allowing the user to carry out at least one of the following steps:
- A step of replacing the presentation displayed in the image viewer with a replacement presentation that corresponds to a presentation assigned to another item of the list shown in the second view but to the same item of the list shown in the first view.

The step of replacing may comprise activating the item to which the replacement presentation was assigned by clicking on the item, by pressing a toolbar item, or by using a keyboard shortcut, for example.

In particular, the replacement presentation corresponds to a presentation assigned to the item arranged in the list shown in the second view precedingly or subsequently to the item to which the presentation to which the presentation displayed in the image viewer is assigned.

In other words, the user can initiate a replacement of the presentation displayed in the image viewer with a replacement presentation that is a presentation of the same study as the presentation to be replaced (this means the presentation currently displayed in the image viewer). The replacement presentation can be assigned to the item that is arranged in the list shown in the second view one position above or one position below the item to which the presentation to be replaced is assigned. This also means that the sequence of replacements may occur in a predefined manner if the list shown in the second view looks always the same.
- A step of replacing the presentation displayed in the image viewer with a replacement presentation that corresponds to a presentation of a study assigned to another item of the list shown in the first view but to the same item of the list shown in the second view.

The step of replacing may comprise activating the item to which the replacement presentation was assigned by clicking on the item, by pressing a toolbar item, or by using a keyboard shortcut, for example.

In particular, the replacement presentation corresponds to a presentation of the study assigned to the item arranged in the list shown in the first view precedingly or subsequently to the item to which the study to which the presentation displayed in the image viewer is assigned.

In other words, the user can initiate a replacement of the presentation displayed in the image viewer with a replacement presentation that is a presentation of the same kind as the presentation to be replaced (this means the presentation currently displayed in the image viewer). The replacement presentation can be assigned to the item that is arranged in the list shown in the first view one position above or one position below the item to which the presentation to be replaced belongs. This also means that the sequence of replacements may occurs in a predefined manner if the list shown in the first view is an ordered always in the same manner.
- A step of switching back and forth between displaying in the image viewer a first presentation and a second presentation, wherein the first presentation is a presentation assigned to a first item in the list shown in the second view and the second presentation is a presentation assigned to a second item in the list shown in the second view. The first presentation and the second presentation are usually assigned to the same item of the list shown in the first view, this means they belong to the same study.

The step of switching back and forth may comprise activating items by clicking on the item, by pressing a toolbar item, or by using a keyboard shortcut, for example.
- A step of switching back and forth between displaying in the image viewer a first presentation and a second presentation, wherein the first presentation is a presentation of the study assigned to a first item in the list shown in the first view and the second presentatin is a presentation of the study assigned to a second item in the list shown in the first view. The first presentation and the second presentation are usually assigned to the same item of the list shown in the second view, this means they are of the same kind of presentations.

The step of switching back and forth may comprise activating items by clicking on the item, by pressing a toolbar item, or by using a keyboard shortcut, for example.

A tool, such as a toolbar item of the user interface or a keyboard shortcut, may be available in the step of switching back and forth according to the above third or forth bullet point. The tool may be configured to perform the replacement of the presentation displayed in image viewer in a toogle way, this means a replacement of the displayed presentation when using the tool and a replacement back to the initially displayed presentation when using the tool again.

Such a "toogle tool" may be used for example to switch back and forth between the current study and the immediate previous study to compare and analyse if a region of interest, such as a medical lesion, is modified in the current study compared to the previous study.

Alternatively or in addition, such a "toogle tool" may be used to switch back and forth between presentation belonging to the same study and having different contrast to assess for example a region of interest or a medical lesion in different contrasts. An important example is, while assessing a medical lesion on the FLAIR contrast, to switch to a contrast-enhanced contrast to verify if the medical lesion uptakes contrast or not, and switch back to the FLAIR contrast to continue the examination of the rest of the presentations.

The invention relates further to a system for at least one of selecting, ordering, displaying and navigating virtual presentations of a body portion, wherein the virtual presentations are generated during medical studies, wherein the studies and virtual presentations may be as disclosed with respect to any embodiment of the method.

The system may be configured or may have components configured to carry out any step of the method in any embodiment disclosed and/or to provide any opportunity to a user in any embodiment disclosed with respect to the method.

Likewise, the method in any embodiment disclosed may comprise any step related to any feature disclosed with respect to the system.

In particular, the invention relates to a system for selecting and displaying presentations generated in medical studies, wherein the system is configured to carry out a method according to any embodiment disclosed with respect to the method for selecting and displaying (and optionaly orderning and/or navigating) virtual presentations of body portions generated in medical studies. Therefore, the system comprises a controller and a user interface. The user interface comprises an image viewer, a first view for a list of studies or an adapted list of studies, a second view for a list of presentations or an adapted list of presentatsions, and tools for selecting an item of the list shown in the first view and an item of the list shwon in the second view.

The user interface may be according to any embodiment of the user interface disclosed with respect to the method. In particular, the user interface may comprise any tool, toolbar items, buttons etc. disclosed with respect to embodiments of the method.

The first and second views as well as the list displayed in the first view and the list displayed in the second view may be according to any embodiment disclosed with respect to the method. In particular, the first and second views as well as the list disclosed in the first view and the list disclosed in the second view may be configured to generate any functionality, in particular any functionality with respect to selecting, switching, navigating and displaying, disclosed with respect to any embodiment of the method.

The controller may be configured to carry out at least one of the step of providing a list of studies in any embodiment disclosed, the step of providing a list of presentations in any embodiment disclosed, the step of assigning studies to an item of the list of studies in any embodiment disclosed, the step of assigning presentations to an item of a list of presentations in any embodiment disclosed, the step of providing the list of studies or an adapted list of studies to the user in any embodiment disclosed, and a step of providing the list of presentations or an adapted list of presentations to the user in any embodiment disclosed.

In particular, the controller may be configured to carry the steps of the method that are automated steps.

Alternatively or in addition, the controller may be configured to update information provided to the user via the user interface, such as the presentation displayed in the image viewer and/or the list provided in the second view or the links to which items of the list shown in the second view refers, in reaction to a user input.

The invention concerns further a computer programm comprising instructions which, when the program is executed by a computer, cause the computer to carry out the method according any embodiment disclosed.

The invention concerns further a computer-readable medium having stored thereon instructions which, when executed by a computer, cause the computer to carry out the method according to any embodiment disclosed.

The invention concerns further a data carrier signal carrying instructions which, when executed by a computer, cause the computer to carry out the method according to any embodiment disclosed.

In particular, the invention concerns any reproducible computer-readable signal encoding the computer program that, when loaded and executed on a computer, causes the computer to carry out the method according to any embodiment disclosed.

The computer that is caused to carry out the method may be a computer or a computer network of the system in any embodiment disclosed.

The invention concerns further a method of manufacturing a non-transitory computer readable medium, comprising the step of storing, on the computer readable medium, computer-executable instructions which when executed by a processor of a computing system, cause the computing system to perform the method according to any embodiment disclosed.

### BRIEF DESCRIPTION OF THE DRAWINGS

Exemplarily embodiments of the invention are shown in the following figures. In the figures, the same reference symbol is used for identical or comparable elements. It shows:
- **Figure 1**: a flow chart of an exemplary embodiment of the method;
- **Figure 2**: an exemplary embodiment of a user interface;
- **Figure 3**: a visualization of an exemplary embodiment of a step of assigning studies to a list of studies;
- **Figure 4**: a visualization of an exemplary embodiment of a step of assigning presentations to a list of presentations;
- **Figure 5**: a visualization of an exemplary embodiment of the steps of assigning studies to a list of studies and of assigning presentations to lists of presentations; and
- **Figure 6**: a visualization of an exemplary embodiment of navigating in the presentations of a plurality of studies and of a plurality of kinds of presentations.

### DETAILED DESCRIPTION OF THE EMBODIMENTS

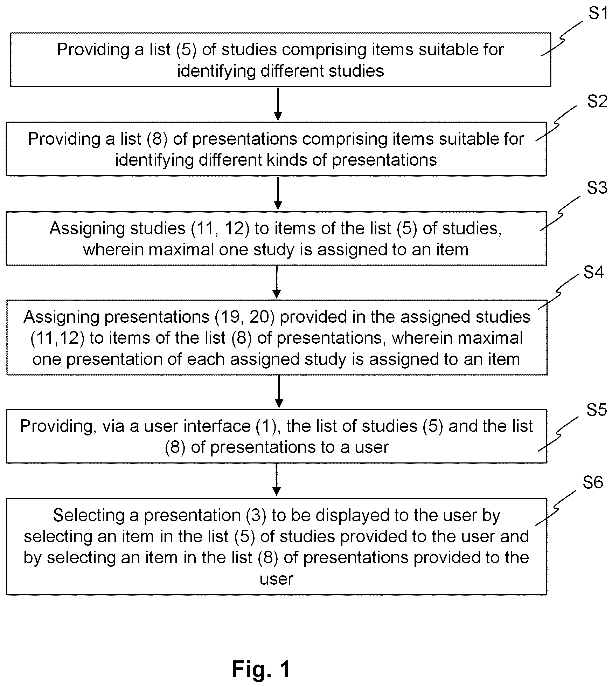

Figure 1 shows a flow chart of an exemplary embodiment of the method for selecting and displaying presentations of a body portion of a patient, such as 3D representations of the body portion. The presentations are received usually from a server/archive on which the presentations after their generation in a medical examination are stored. For example, the archive is a picture archiving communication system (PACS).

Alternatively, the presentations of the body portion may be received from any other server/archive, for example from the imaging system used for acquiring the presentations or raw data used to generate the presentations, or from any sever that can be in communication with the imaging system and the system used for carrying out the method.

The exemplary embodiment shown in Figure 1 comprises:
- A step S1 of providing a list 5 of studies comprising items suitable for identifying the stored results of different studies.

Items that indicate by their labeling the date a study was carried out are good examples of such items. Hence, the list of studies may be a list of dates, for example.

The list 5 of studies is usually a chronological list.
- A step S2 of providing a list 8 of presentations comprising items suitable for identifying different kinds of presentations.

Items that indicate by their labeling the contrast that is inherent to a kind of presentations due the manner the presentations of the kind of presentations are generated are good examples of such items. Hence, the list of presentations may be a list of contrasts, for example.

The list 8 of presentations is usually an organized list. For example, it is organized according to a workflow used by a practitioner during a medical evaluation of the body portion.
- A step S3 of assigning studies 11, 12 to items of the list 5 of studies, wherein maximal one study is assigned to an item.

For example, every study that refer to the body portion of the patient, said body portion being the body portion to be evaluated, may be assigned each to exactly one item of the list 5 of studies but never to an item of the list 5 of studies to which another study is assigned.

It goes without saying that the study that is assigned to an item of the list 5 of studies is the study that corresponds to, for example has, the feature (characteristic) indicated by item to make the item suitable for identifying different studies.
- A step S4 of assigning presentations 19, 20 to items of the list 8 of presentations, wherein the presentations belong to studies 11, 12 assigned to items of the list 5 of items and wherein maximal one presentation of each assigned study is assigned to an item of the list 8 of presentations.

It goes without saying that the presentations that are assigned to an item of the list 8 of presentation are presentations of the kind to which the item refers. In other words, the presentations that are assigned to an item of the list 8 of presentation have the feature (characteristic) that corresponds to the feature (characteristic) indicated by the item to make the item suitable for identifying different kinds of presentations.

For example, for each study assigned to an item of the list 5 of studies all presentations may be assigned to exactly one item of the list 8 of presentations but never to an item of the list 8 of presentations to which another presentation of the same study is assigned.
- A step S5 of providing, via a user interface 1, the list 5 of studies and the list 8 of presentations to a user, wherein the user interface allows for the selection of one item in the provided list 5 of studies and of one item in the list 8 of presentations, wherein the selections can be changed.

Further, the user interface 1 is configured to display the presentation belonging to the study selected by selecting an item in the provided list 5 of studies and being of the kind selected by selecting an item in the provided list 8 of presentations.
- A step S6 of selecting a presentation 3 to be displayed to the user by selecting an item in the list 5 of studies provided to the user and by selecting an item in the list 8 of presentations provided to the user.

In particular, the user selects in a consecutive manner the presentations she/he wants to see by selecting items in the provided list 5 of studies and/or in the provided list 8 of presentations. In other words, the user navigates in a very simple and intuitive manner through the presentations available for the body portion of the patient to be evaluated.

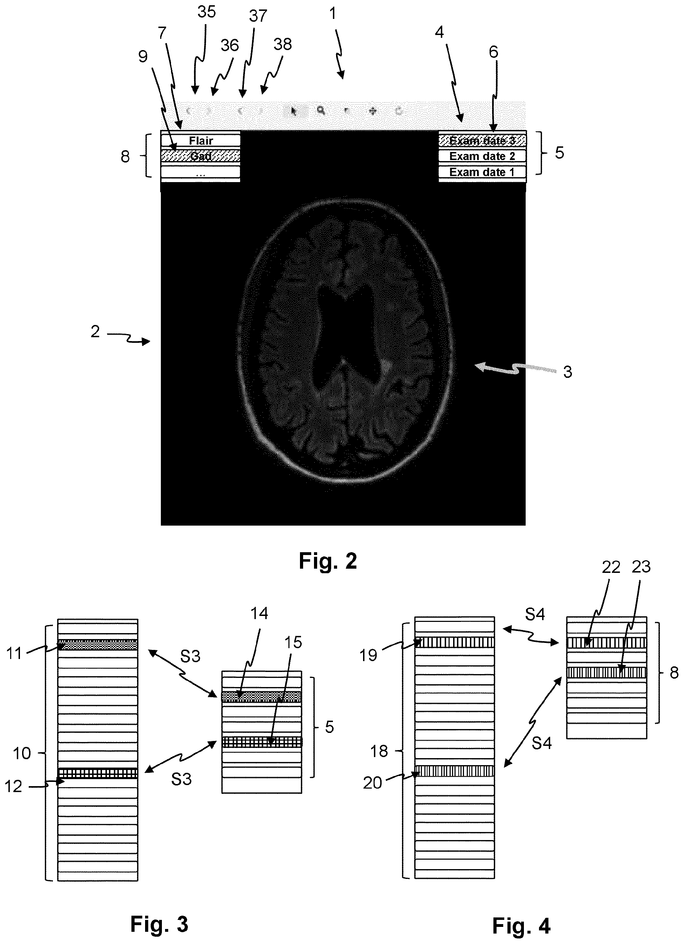

**Figure 2** shows an exemplary embodiment of a user interface 1 that is configured for use in the method, for example in the method as disclosed with respect to figure 1.

The user interface 1 comprises an image viewer 2, in which the presentation selected by selecting an item in the provided list 5 of studies and an item in the provided list 8 of presentations is displayed (displayed presentation 3).

The user interface 2 comprises further a first view 4 in which the list 5 of studies is provided and a second view 7 in which the list 8 of presentations is provided.

First and second views are usually active views in the sense that an item of the lists shown in the views can be selected by clicking on it or by touching it, for example.

The first and second are usually further configured to highlight the currently selected study 6 and the currently selected kind of presentation 9.

The user interface 1 has usually further tools that allow easy and intuitive navigation through the presentations available for the body portion of the patient to be evaluated, for example toolbar buttons, such as a toolbar button 35 to display the presentation assigned to one back (with respect to the currently selected kind of presentation 9) in the list 7 of presentations (and belonging to currently selected study 6), a toolbar button 36 to display the presentation assigned to one forwards (with respect to the currently selected kind of presentation 9) in the list 7 of presentations (and belonging to currently selected study 6), a toolbar button 37 to display the presentation corresponding to one back (with respect to the currently selected study 6) in the list 5 of studies (and being of the currently selected kind of presentation 9), and a toolbar button 38 to display the presentation corresponding to one forwards (with respect to the currently selected study 6) in the list 5 of studies (and being of the currently selected kind of presentation 9).

**Figure 3** shows a visualization of an exemplary embodiment of the step of assigning studies to the list 5 of studies.

According to the embodiment of figure 3, all studies 10 of a patient, or all studies 10 of the patient concerning a specific body portion, are accessed and the studies that correspond to an item of the list 5 of studies are assigned to the corresponding item as disclosed with respect to figure 1.

In a variant of the method, the list 5 of studies is provided in an automated manner by accessing all studies of a patient and by identifying the studies of interest for the medical condition of the patient to be assessed. For example, all studies of a specific body portion of the patient and/or all studies having been generated in a given period of time are identified and items of the list 5 of studies are generated, said items being suitable for identifying the study that has initiated the generation of said item. Again, the labelling of the item may comprise the date of the study.

Figure 3 shows schematically how, in a step S3 of assigning studies to the list 5 of studies, a first study 11 of all studies 10 is assigned to the corresponding item 14 of the list 5 of studies and how a second study 12 of all studies 10 is assigned to the corresponding item 15 of the list 5 of studies.

**Figure 4** shows a visualization of an exemplary embodiment of the step of assigning presentations to the list 8 of presentations.

According to the embodiment of figure 4, all presentations 18 of a study that is assigned to an item of the list 5 of studies are accessed and the presentations that correspond to an item of the list 8 of presentations are assigned to the corresponding item as disclosed with respect to figure 1.

In a variant of the method, the list 8 of presentations is provided in an automated manner by accessing all studies assigned to an item of the list 5 of studies and by identifying the kind of presentations available in the studies. An item of the list 8 of presentations is then generated for each identified kind of presentation. Again, the labelling of the item may comprise a hint towards the contrast of the kind of presentation related to the item.

Figure 4 shows schematically how, in a step S4 of assigning presentations to the list 8 of presentations, a first presentation 19 of all presentations 18 is assigned to the corresponding item 22 of the list 8 of presentations and how a second presentation 20 of all presentations 18 is assigned to the corresponding item 23 of the list 8 of presentations.

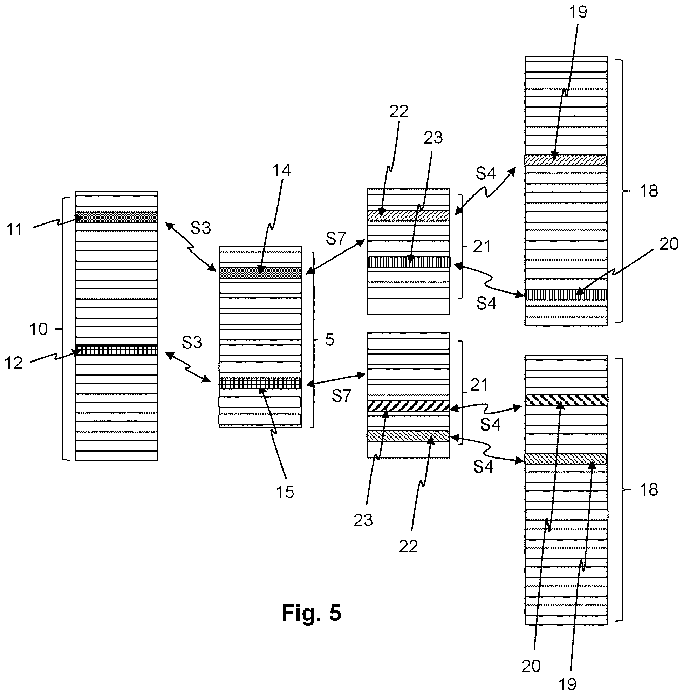

**Figure 5** shows a visualization of an exemplary embodiment of the steps of assigning studies to the list 5 of studies and of assigning presentations to lists 21 of presentations.

The embodiment the step S3 of assigning studies and of the step S4 of assigning presentations according to figure 5 distinguishes from the embodiments of these steps shown in figures 3 and 4 in that a list 21 of presentations is provided for each study 11, 12 assigned to an item of the list 5 of studies. In other words, the method according to figure 5 comprises a step S7 of providing a list 21 of presentations for each study assigned to an item of the list 5 of studies.

The lists 21 of presentations provided in said step S7 may be as the list 5 of presentations disclosed with respect to figures 1-4.

The step S7 of providing a list 21 of presentations for each study assigned to an item of the list 5 of studies may be an automated step. For example, the lists 21 of presentations may be generated by accessing the assigned studies and by identifying the kind of presentations available in the studies.

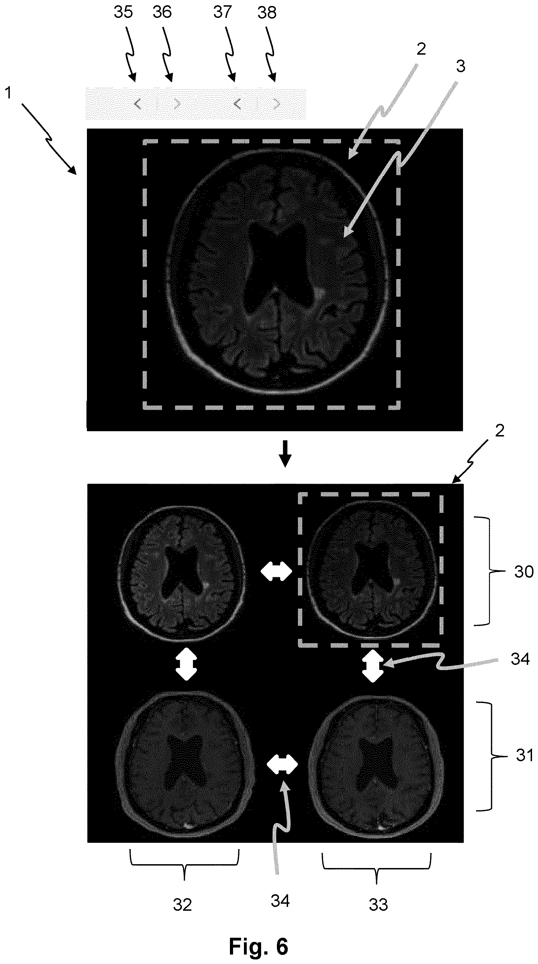

**Figure 6** shows a visualization of an exemplary embodiment of navigating in the presentations of a plurality of studies and of a plurality of kinds of presentations.

The upper part of figure 6 shows a selection of elements of the user interface 1 shown in figure 2. In particular, the image viewer 2 in which the displayed presentation 3 is arranged and the toolbar buttons 35-38 for navigating back and forth in the list 5 of studies provided in the first view 4 and the list 8 of presentations provided in the second view 7 are shown.

The lower part of figure 6 visualizes how a presentation of a specific study and being of a specific kind of presentation is displayed in the image viewer 2 by one-click or one-key operations 34.

Each presentation assigned to an item of the list 8 of presentation is also assigned, via the study to which it belongs, to an item of the list 5 of studies. This makes a clear identification, and hence display in the image viewer 2, of each presentation assigned to an item of the list 8 of presentation possible.

For visualization, figure 6 shows two presentations 30 assigned to a first item in the list 8 of presentations and two presentations 31 assigned to a second item in the list 8 of presentations. One of the presentations 30 assigned to the first item and one presentation assigned to the second item are presentations 32 assigned further to a first item in the list 5 of studies. The other presentation of the presentations 30 assigned to the first item of the list 8 of presentations and the other presentation of the presentations 31 assigned to the second item of the list 8 of presentations are presentations 33 assigned further to a second item in the list 5 of studies.

### Reference signs

- 1.: user interface
- 2.: image viewer
- 3.: displayed presentation
- 4.: first view (view for the list of studies)
- 5.: list of studies
- 6.: selected study
- 7.: second view (view for the list of series)
- 8.: list of presentations
- 9.: selected kind of presentation
- 10.: all studies of one patient
- 11.: one study
- 12.: another study
- 14.: an item of the list of studies to which a study is assigned
- 15.: another item of the list of studies to which another study is assigned
- 18.: all presentations of one study
- 19.: one presentation
- 20.: another presentation
- 21.: list of presentations of a corresponding study
- 22.: an item of the list of presentations to which a presentation is assigned
- 23.: another item of the list of presentations to which another presentation is assigned
- 30.: presentations corresponding to one item of the list of presentations
- 31.: presentations corresponding to another item of the list of presentations
- 32.: presentations corresponding to one item of the list of studies
- 33.: presentations corresponding to another item of the list of studies
- 34.: 1-click / 1-touch / 1-key operation
- 35.: toolbar button to display the presentation corresponding to one back in the list of presentations
- 36.: toolbar button to display the presentations corresponding to one forward in the list of presentations
- 37.: toolbar button to display the presentation corresponding to one back in the list of studies
- 38.: toolbar button to display the presentations corresponding to one forward in the list of studies

- S1: Providing a list of studies
- S2: Providing a list of presentations
- S3: Assigning a study to an item of the list of studies
- S4: Assigning a presentation to an item of the list of presentations
- S5: Providing the list of studies and the list of presentations to a user
- S6: Selecting a presentation by selecting an item of the provided list of studies and an item of the provided list of presentations
- S7: Providing a list of presentations for each study assigned to an item of the list of studies

## Claims

1. A computer-implemented method for selecting and displaying virtual presentations of a body portion generated in medical studies, the method comprises:
• A step (S1) of providing a list (5) of studies comprising at least one item that is characteristic for a kind of study;
• A step (S2) of providing a list (8) of presentations comprising at least one item that is characteristic for a kind of presentation;
• A step (S3) of assigning a study to an item of the at least one item of the list of studies;
• A step (S4) of assigning a presentation provided in the study assigned to an item of the at least one item of the list of studies to an item of the at least one item of the list of presentations;
• A step (S5) of providing to a user via a user interface (1) the list (5) of studies or an adapted list of studies and the list (8) of presentations or an adapted list of presentations,
wherein the user interface comprises an image viewer (2), a first view (4) for the list (5) of studies or the adapted list of studies, and a second view (7) for the list (8) of presentations or the adapted list of presentations;
• A step (S6) of selecting a presentation (3) to be displayed in the image viewer (2) by selecting an item of the list shown in the first view (4) and by selecting an item of the list shown in the second view (7).

2. The method according to claim 1, wherein the items of the list (8) of presentations are suitable for identifying different presentations by the items referring to settings available or used during acquisition of raw data or presentations and/or by the items referring to features available or used during processing of acquired raw data or acquired presentations.

3. The method according to claim 1 or 2, wherein the list of studies comprises unique items only and the list of presentations comprises unique items only.

4. The method according to one of claims 1-3, wherein the list shown in the first view (4) is an ordered list, and the list shown in the second view (7) is an ordered list.

5. The method according to one of claims 1-4, wherein the items of the list shown in the second view (7) are linked to the assigned presentations of the study that is assigned to the selected item (6) of the list shown in the first view (4).

6. The method according to one of claims 1-5, comprising a step (S7) of providing a list of presentations for each study assigned to an item of the at least one item of the list (5) of studies, wherein the presentations of an assigned study or a selection of the presentations of the assigned study are assigned to the list of presentations provided for said study, and wherein the list shown in the second view (7) is the list (8) of presentations or the adapted list of presentations that corresponds to the study assigned to the selected item (6) of the list shown in the first view (4).

7. The method according to one of claims 1-6, wherein the selection of another item of the list shown in the first view (4) than the currently selected item (6) occurs at a fixed item in the list shown in the second view (7), and the selection of another item of the list shown in the second view (7) than the currently selected item (9) occurs at a fixed item in the list shown in the first view (4).

8. The method according to one of claims 1-7, wherein the presentations assigned to an item of the list of presentations are coregistered to one another, to one of the presentations, or to a reference presentation.

9. The method according to claim 8, wherein the presentations assigned to an item of the list of presentations are coregistered to a reference presentation and wherein an algorithm determines the reference presentation from a set of presentations that comprises all presentations assigned to an item of the list of presentations.

10. The method according to one of claims 1-9, wherein the user interface (1) comprises tools (35-38) allowing the user to at least one of:
• Replace the presentation (3) displayed in the image viewer (2) with a replacement presentation that corresponds to the presentation assigned to the item arranged in the list shown in the second view (7) precedingly or subsequently to the item to which the presentation (3) displayed in the image viewer is assigned;
• Replace the presentation (3) displayed in the image viewer (2) with a replacement presentation that belongs to the study assigned to the item arranged in the list shown in the first view (4) precedingly or subsequently to the item to which the study is assigned to which the presentation (3) displayed in the image viewer belongs;
• Switch back and forth between displaying in the image viewer (2) a first presentation and a second presentation, wherein the first presentation is a presentation assigned to a first item in the list shown in the second view (7) and the second presentation is a presentation assigned to a second item in the list shown in the second view (7);
• Switch back and forth between displaying in the image viewer (2) a first presentation and a second presentation, wherein the first presentation is a presentation of the study assigned to a first item in the list shown in the first view (4) and the second presentation is a presentation of the study assigned to a second item in the list shown in the first view (4).

11. A system for selecting and displaying virtual presentations of a body portion generated in medical studies, comprising a controller and a user interface (1), wherein the user interface comprises an image viewer (2), a first view (4) for a list (5) of studies or an adapted list of studies, a second view (7) for a list (8) of presentations or an adapted list of presentations, and tools (35-38) for selecting an item of the list shown in the first view (4) and an item of the list shwon in the second view (7), and wherein the system is configured to carry out a method according to one of claims 1-10.

12. A computer programm comprising instructions which, when the program is executed by a computer, cause the computer to execute the steps of the method according to one of claims 1-10.

13. A reproducible computer-readable signal encoding the computer program according to claim 12.
